# EUROPEAN PATENT APPLICATION

(11) **EP 3 831 447 A1**
(43) Date of publication of application: **09.06.2021**
(21) Application number: 19843694.1
(22) Date of filing: 01.08.2019
(51) Int. Cl.: A61N 5/06

(54) **DEVICE AND METHOD FOR CONTROLLING BRAIN WAVES AND CELL ACTIVITY BY PHOTIC STIMULATION, AND DEVICE FOR IMPROVEMENT, PROPHYLAXIS, OR ENHANCEMENT OF BRAIN FUNCTION**

(30) Priority: 01.08.2018 JP 2018145270
(71) Applicant: Tsubota Laboratory, Inc., Tokyo 160-0016 (JP)
(72) Inventor: HAYANO, Motoshi, Tokyo 160-0016 (JP); TSUBOTA, Kazuo, Tokyo 160-0016 (JP)
(74) Representative: Mewburn Ellis LLP
(86) International application number: PCT/JP2019/030384
(87) International publication number: WO 2020/027305

(57) **Abstract**

To provide a brain wave and cell activity control device and method based on light stimulation in which light having a specific wavelength, such as violet light, is irradiated using continuous light or a specific blinking frequency, and a device for improvement, prevention, or increase in brain function. The above-described problem is solved by a brain wave and cell activity control device, based on light stimulation, that controls brain waves or cell activity by irradiating light having a specific wavelength onto a subject using continuous light or a specific blinking frequency. The brain wave and cell activity control device comprises a light source that irradiates the light having a specific wavelength using continuous light or a specific blinking frequency, and a control unit that controls an emission of light that induces specific brain waves, the brain waves of the subject having received the light being the same or substantially the same as, or different from a frequency of the light in an irradiation state. At this time, the light is preferably violet light, and the irradiation state of the light is preferably continuous light or a blinking frequency greater than 0 Hz and less than or equal to 150 Hz.

## Description

### Field of the Invention

The present invention relates to a brain wave and cell activity control device and method based on light stimulation in which light having a specific wavelength, such as violet light, is irradiated at a specific blinking frequency, and a device for improvement, prevention, or increase in brain function.

### BACKGROUND ART

In recent years, the effects of light on a human body have been studied from various perspectives and reported based on new findings. For example, it has been reported that circadian rhythm is improved by exposure to sunlight (Non-Patent Document 1), light emitted from light-emitting diode (LED) lighting, a liquid crystal display that uses an LED as a backlight, or the like has a significant effect on the body and mind (Non-Patent Document 2), violet light prevents myopia and suppresses an onset of myopia (Patent Document 1), and the like. In particular, the present inventors have recently made an interesting report on the effects of violet light on the eye. For example, in Patent Document 1 and Non-Patent Document 7, it is proposed that light having a specific wavelength is effective in myopia prevention and myopia suppression, and there are great expectations in recent years with the number of persons with myopia still increasing worldwide.

Further, research and development on various treatment techniques have also been active. In particular, research and development on treatment techniques that can eliminate or reduce the use of drugs and thus reduce the burden on the body are underway. The present inventors are conducting research and development on the application of light having a specific wavelength, such as violet light, to treatment. As an example thereof, a non-invasive cornea and sclera strengthening device and method that solve the problems of conventional invasive corneal crosslinking in which the corneal epithelium is ablated and ultraviolet A (UVA) is irradiated at a high irradiance, enabling treatment in daily life without treatment in a confined state at a medical institution for a fixed period of time, and without ablation of the corneal epithelium have been proposed (unpublished patent).

It should be noted that, in Patent Document 2 and Non-Patent Document 4, it is reported that, in a study using mice with Alzheimer's disease, when the synchronization of gamma wave vibration is restored in the brain, the amyloid β protein accumulated in the brain is removed.

### PRIOR ART DOCUMENTS

### Non-Patent Documents

Non-Patent Document 1: Megumi Hatori, Kazuo Tsubota, Anti-Aging Medicine - Journal of Japanese Society of Anti-Aging Medicine, Vol. 11, No. 3, 065(385)-072(392), (2015)
Non-Patent Document 2: Kazuo Tsubota, "Blue Light - Threat to Internal Clock," Shueisha (November 20, 2013)
Non-Patent Document 3: Hidemasa Torii et al., EBioMedicine, "DOI: http://dx.doi.org/10.1016/j.ebiom.2016.12.007"
Non-Patent Document 4: NATURE, Vol. 540, 8, December 2016, pp. 231 to 235 Patent Documents

Patent Document 1: WO 2015/186723 A1
Patent Document 2: US 2017/0304584 A1

### SUMMARY OF THE INVENTION

### Problems to be Solved by the Invention

The present invention is a result obtained by the inventors finding that irradiation of violet light at a blinking frequency affects brain waves and further conducting studies on the basis of this finding, and an object of the present invention is to provide a brain wave and cell activity control device and method based on light stimulation in which light having a specific wavelength, such as violet light, is irradiated using continuous light or a specific blinking frequency.

Further, another object of the present invention is to provide a device that irradiates violet light or white light using continuous light or a specific blinking frequency to improve or prevent depression, suppress or prevent stress, improve or enhance concentration, improve or prevent Alzheimer's disease, and improve or prevent cognitive functions, in other words, to provide a device for improvement, prevention, or increase in brain function.

### Means for Solving the Problems

(1) A brain wave and cell activity control device based on light stimulation according to the present invention is a device that controls brain waves or cell activity by irradiating light having a specific wavelength onto a subject using continuous light or a specific blinking frequency, and comprises a light source that irradiates the light having a specific wavelength using continuous light or a specific blinking frequency, and a control unit that controls an emission of light that induces specific brain waves, the brain waves of the subject having received the light being the same or substantially the same as, or different from a frequency of the light in an irradiation state.

According to this invention, the brain waves of the subject who receives the light become the same or substantially the same as a frequency of the light in the irradiation state (continuous light or specific blinking frequency), making it possible to apply various stimulation to the brain or enhance the cell activity in the body by controlling the irradiation state of the light. In particular, when a blinking frequency or the like is irradiated, it is possible to induce specific brain waves that are the same or substantially the same as, or different from a frequency of the irradiated light in the irradiation state, and thus control the stimulation state with respect to the brain and extend application to various mind and body improvement effects and treatment effects arising therefrom.

In the brain wave and cell activity control device according to the present invention, the light is violet light. According to this invention, it is possible to irradiate violet light having a wavelength outside the visible light region onto the subject, and thus induce specific brain waves that are the same or substantially the same as, or different from the blinking frequency of the light without sensation of flicker or glare such as with white light. It should be noted that violet light is wavelength light of 360 to 400 nm, and that wavelength light has low visual sensitivity compared to that of white light and is in a wavelength region in which the subject does not feel or is less likely to feel discomfort.

In the brain wave and cell activity control device according to the present invention, the irradiation state of the light is continuous light or a blinking frequency greater than 0 Hz and less than or equal to 150 Hz. According to this invention, specific brain waves that are the same or substantially the same as, or different from a frequency of the light in such an irradiation state can be induced.

In the brain wave and cell activity control device according to the present invention, the light is irradiated at an irradiance within a range of 0.5 to 1000 µW/cm². According to this invention, it is possible to irradiate violet light or the like within the above-described range of irradiance, and thus control the frequency of the brain waves and a generation site thereof as desired. The occurrence of the above-described characteristic phenomenon has been confirmed even with a particularly slight amount of weak light (light having weak light sensitivity), and an effect on the brain and application to cell activity (including gene expression control as well) can be expected.

In the brain wave and cell activity control device according to the present invention, the control unit changes and executes irradiation conditions of the light, such as the irradiation state (including continuous light or blinking frequency), an irradiance, an irradiation time, an irradiation start time, an irradiation end time, and continuous light or blinking frequency, by transmission and reception with an isolation controller such as a mobile terminal. According to this invention, the various irradiation conditions described above are isolated and controlled, making it possible to set irradiation conditions suitable for producing desired brain waves and cell activity as desired to obtain a desired effect. Furthermore, this makes it possible to measure and evaluate how irradiation of a specific wavelength light at a blinking frequency or the like affects brain waves and cell activity and to what extent the irradiation affects the mind and body, and put the results to practical use.

In the brain wave and cell activity control device according to the present invention, preferably the light source is a light source installed in front of or near a face, such as eyeglasses with a light source, a desk-top light source, or a light source mounted on a mobile terminal. According to this invention, it is possible to irradiate a specific light from a light source installed in front of or near the face, such as eyeglasses with a light source that are easy to wear and do not cause discomfort on a daily basis, and thus increase practicality and continuously irradiate the light in various situations and environments as well.

In the brain wave and cell activity control device according to the present invention, the light source may be a non-installation-type light source such as a portable light source, or an installation-type light source such as indoor lighting, a table lamp, or a dedicated device. According to this invention, the device can have various light source forms in accordance with the usage environment.
(2) A brain wave function control method based on light stimulation according to the present invention is a method of controlling brain waves or cell activity by irradiating light having a specific wavelength onto a subject using continuous light or a specific blinking frequency, and comprises controlling an emission of light that induces specific brain waves, the brain waves of the subject having received the light being the same or substantially the same as, or different from the continuous light or the specific blinking frequency.
(3) A device for improvement, prevention, or increase in brain function according to the present invention is a device that improves, prevents, or increases brain function by irradiating violet light or white light onto a subject using continuous light or a specific blinking frequency, and comprises a light source that emits the violet light or the white light, a light-emitting cycle control unit that sets the violet light or the white light to continuous light or a specific blinking frequency, and a light-emitting time control unit that irradiates the violet light or the white light for a specific time or a specific period. The device is used for one or two or more purposes selected from improvement or prevention of depression, suppression or prevention of stress, improvement or increase in concentration, improvement or prevention of Alzheimer's disease, improvement of sleep, and the like.
(4) A device for improvement or prevention of brain function according to the present invention is a device that improves or prevents cognitive functions by irradiating violet light or white light onto a subject using continuous light, and comprises a light source that emits the violet light or the white light, and a light-emitting time control unit that irradiates the violet light or the white light for a specific time or a specific period.

### Effect of the Invention

According to the present invention, it is possible to provide a brain wave and cell activity control device and method in which light having a specific wavelength, such as violet light, is irradiated using continuous light or a specific blinking frequency, producing specific brain waves in the subject that are the same or substantially the same as, or different from a frequency of the light in an irradiation state. In particular, the present invention is characteristic in that specific brain waves in the subject having received the light that are the same or substantially the same as, or different from a frequency of the light in the irradiation state can be induced, and various stimulation can be applied to the mind, body, and brain.

According to the present invention, it is possible to improve or prevent depression, suppress or prevent stress, improve or increase concentration, improve or prevent Alzheimer's disease, improve or prevent cognitive functions, improve sleep, and the like by a device that irradiates violet light or white light onto a subject.

### BRIEF DESCRIPTION OF THE DRAWINGS

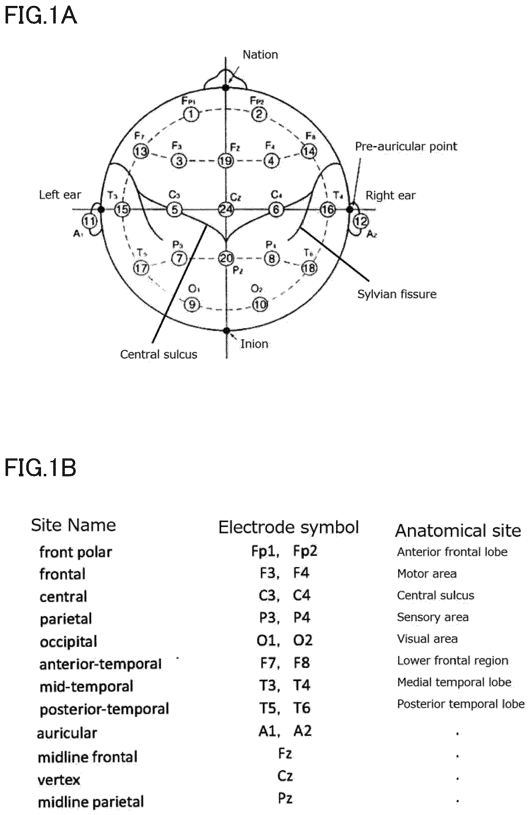

Figs. 1A and 1B constitute an explanatory view of each component of a brain.
Fig. 2A is a result showing generation of a 10-Hz wave in an occipital lobe by irradiation of violet light having a blinking frequency of 10 Hz, and Fig. 2B is a result showing generation of a 60-Hz wave in the occipital lobe by irradiation of violet light having a blinking frequency of 60 Hz.
Fig. 3A is a result showing generation of a 10-Hz wave in a frontal lobe by irradiation of violet light having a blinking frequency of 10 Hz, and Fig. 3B is a result showing generation of a 60-Hz wave in the frontal lobe by irradiation of violet light having a blinking frequency of 60 Hz.
Fig. 4A is a result showing non-generation of a 60-Hz wave in the frontal lobe by irradiation of violet light having a blinking frequency of 10 Hz, and Fig. 4B is a result showing non-generation of a 10-Hz wave in the frontal lobe by irradiation of violet light having a blinking frequency of 60 Hz.
Fig. 5 is an example of violet light eyeglasses that irradiate violet light.
Fig. 6 is a graph showing the relationship between wavelength and spectral irradiance of light of a violet fluorescent lamp.
Fig. 7 is a light spectrum of an LED having a peak wavelength of 375 nm.
Fig. 8 is an explanatory view of typical brain waves.
Figs. 9A to 9C are analysis results of factors contributing to sleep quality.
Figs. 10A and 10B are analysis results of factors that decrease sleep quality.
Figs. 11A and 11B are analysis results of other factors contributing to sleep quality.
Fig. 12A is an evaluation result of inducing depression by using lipopolysaccharide and improving depression by VL 40-Hz frequency stimulation, and Fig. 12B is an evaluation result of inducing depression by using CUMS and improving depression by VL continuous or 40-Hz frequency stimulation.
Fig. 13A is an explanatory view of a left prefrontal cortex Fp1 (international 10-20 system) serving as a measurement point, and Fig. 13B is an explanatory view given a measurement time of 30 minutes (1800 seconds) and a rest time of one minute before and after the measurement time.
Fig. 14 is a test result of significant difference in power spectra.
Figs. 15A to 15C are results showing stress suppression effects by 40-Hz frequency stimulation.
Fig. 16 is a graph showing an average value of stress values for stimulation under each condition.
Figs. 17A and 17B are results showing a decrease in phosphorylated tau by 40-Hz frequency stimulation.
Figs. 18A and 18B are results showing a decrease in phosphorylated tau by 40-Hz frequency stimulation similar to those of Figs. 17A and 17B.
Fig. 19 is an evaluation result of fear memory performed by a CFC test.
Fig. 20 is a result of evaluating spatial memory by Barnes maze after applying white light continuous stimulation or VL continuous stimulation.
Fig. 21 is a result of evaluating activity by using a running wheel after applying VL continuous stimulation.
Figs. 22A to 22C are graphs showing changes in gene expression to which VL continuous stimulation is applied.
Figs. 23A and 23B are graphs showing other changes in gene expression to which VL continuous stimulation is applied.
Figs. 24A to 24C are graphs showing other further changes in gene expression to which VL continuous stimulation is applied.

### Embodiments of the Invention

A brain wave and cell activity control device and method based on light stimulation, and a device for improvement, prevention, or increase in brain function according to the present invention will now be described with reference to the drawings. The present invention is not limited to the contents of the following embodiments and examples, and includes various modifications and applications within the scope of the gist of the present invention.

### [Brain Wave and Cell Activity Control Device Based on Light Stimulation]

A brain wave and cell activity control device based on light stimulation according to the present invention is a device that controls brain waves or cell activity by irradiating light having a specific wavelength onto a subject using continuous light or a specific blinking frequency, and includes a light source that irradiates the light having a specific wavelength using continuous light or a specific blinking frequency, and a control unit that controls an emission of light that induces specific brain waves, the brain waves of the subject having received the light being the same or substantially the same as, or different from a frequency of the light in an irradiation state. Further, a brain wave function control method based on light stimulation is also a method of controlling brain waves or cell activity by irradiating light having a specific wavelength onto a subject using continuous light or a specific blinking frequency, and includes controlling an emission of light that induces specific brain waves, the brain waves of the subject having received the light being the same or substantially the same as, or different from a frequency of the light in an irradiation state.

This brain wave and cell activity control device induces specific brain waves, the brain waves of the subject having received the light being the same or substantially the same as, or different from a frequency of the light in the irradiation state, making it possible to apply various stimulation to the brain or enhance the cell activity in the body by controlling the irradiation state of the light. In particular, when a blinking frequency or the like is irradiated, it is possible to produce specific brain waves that are the same or substantially the same state as a frequency of the irradiated light in the irradiation state, or different from a frequency of the light in the irradiation state, and thus control the stimulation state with respect to the brain and extend application to various mind and body improvement effects and treatment effects arising therefrom. It should be noted that sound, vibration, a magnetic field, an electric field, or the like can also be applied along with the irradiation of the light of such a blinking frequency or the like in the irradiation state, and a combined action of both can be produced.

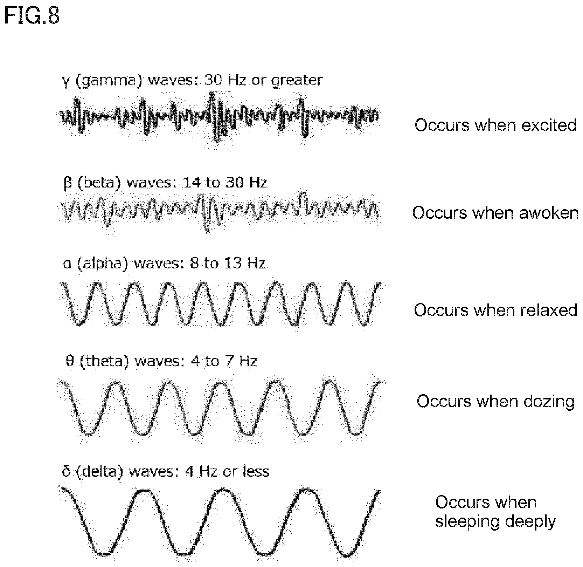

Fig. 8 shows generally understood brain wave classifications. Electrical vibrations (brain waves) are produced in the brain via neural circuits, and such brain waves are generally classified into delta waves of approximately 4 Hz or less, theta waves of approximately 4 to 7 Hz, alpha waves of approximately 8 to 13 Hz, beta waves of approximately 14 to 30 Hz, and gamma waves of approximately 30 Hz or greater.

In the experiment described later, the blinking frequencies of 10 Hz and 60 Hz are tested as examples, and the ranges can be said to be those of alpha waves and gamma waves. In general, alpha waves are referred to as brain waves that occur when the mind and body are relaxed, and gamma waves are referred to as brain waves generated when the mind and body are excited. In the present invention, it is possible to produce brain waves in the subject that are the same or substantially the same frequency as the blinking frequency of the light with such brain waves irradiated. Further, it is also possible to induce different specific brain waves.

The present invention has a remarkable characteristic in that brain waves having the same or substantially the same frequency as or brain waves having a specific frequency different from the blinking frequency of the irradiated light can be induced, and the generation of such brain waves is controlled by light irradiation conditions, making it possible to impart mental and physical actions based on the brain waves described above, such as, for example, emotion, motivation, memory, concentration, relaxation, mood elevation, awakening, sleep, sleepiness, sleep introduction, and dreaming, to the subject. Even at the present time, human experiments have obtained results in various ways, such as sleep improvement, relaxation, and dreaming (dreaming is effective in memory formation immediately before non-rapid eye movement sleep (NREM) sleep). Furthermore, it is highly expected that the present invention will act on cell activity and affect various mental and physical actions (including treatment effects), such as brain wave-based cell activity action and disease action such as, for example, changes in intracerebral transmitters, changes in body secretions, effects on proteins, Alzheimer's disease, brain dysfunction, age-related retinal macular degeneration, dependence, depression, dissociative disorder, obsessive-compulsive disorder, sleep disorder, eating disorder, bipolar disorder (manic depression), adjustment disorder, schizophrenia, dementia, personality disorder, developmental disorder, panic disorder, post-traumatic stress disorder (PTSD), gender identity disorder, and epilepsy.

### [Generation of Brain Waves by Light Stimulation]

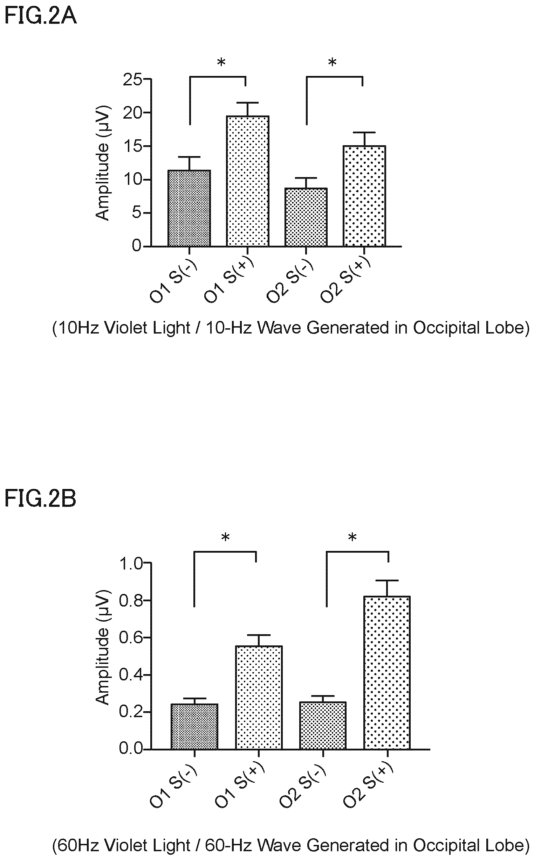

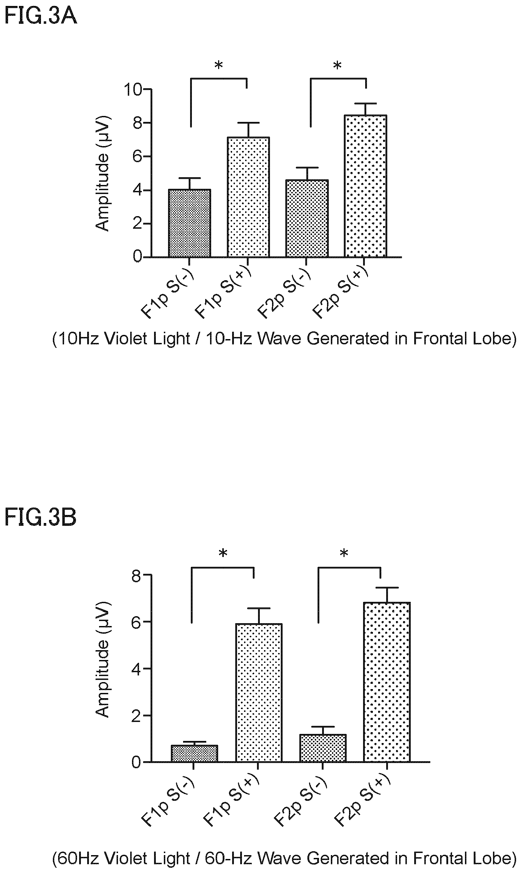

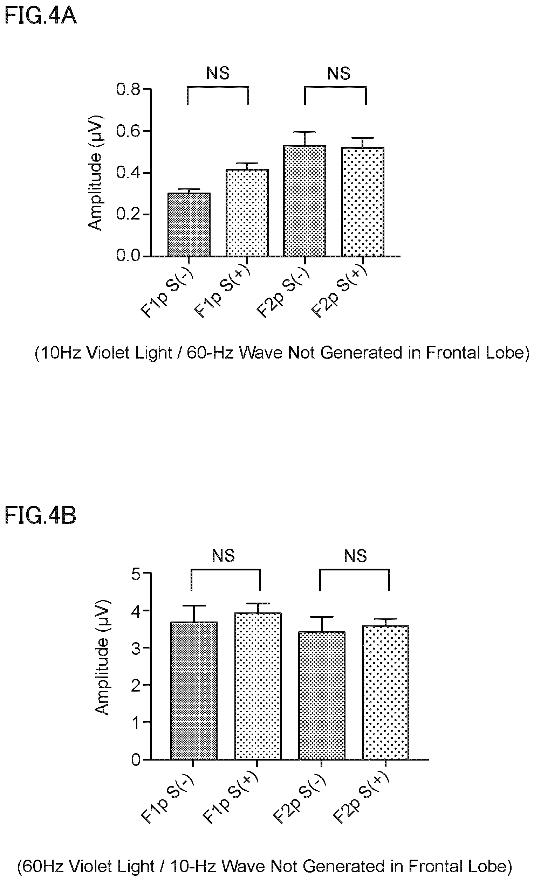

Figs. 2A and 2B, Figs. 3A and 3B, and Figs. 4A and 4B are results obtained by measuring brain waves when light is irradiated in a blinking manner. As shown in Figs. 2A and 3A, irradiation of violet light at a blinking frequency of 10 Hz yielded, as a result, generation of a 10-Hz wave in the occipital lobe and the frontal lobe. Further, as shown in Figs. 2B and 3B, irradiation of violet light at a blinking frequency of 60 Hz yielded, as a result, generation of a 60-Hz wave in the occipital lobe and the frontal lobe. On the other hand, in Figs. 4A and 4B, irradiation of violet light at a blinking frequency of 10 Hz did not generate a 60-Hz wave in the frontal lobe, and irradiation of violet light at a blinking frequency of 60 Hz did not also generate a 10-Hz wave in the frontal lobe. These results show an extremely surprising phenomenon that brain waves having the same or substantially the same frequency as the blinking frequency of the irradiated light were generated. Moreover, in this experiment, violet light, which is weak light (low irradiance), non-glaring, and minimally disturbing compared to white light, is irradiated in a blinking manner, and thus results have been obtained showing that the glare, disturbing feel, and flicker sensation that often occur with visible light such as white light are non-existent, and, in particular, by raising the blinking frequency to the extent that blinking is not a concern, the present invention offers sufficient practicality for use in daily life.

The light source, irradiation conditions, brain wave measurements, and the like used in the measurements of Figs. 2A and 2B, Figs. 3A and 3B, and Figs. 4A and 4B will be described below.

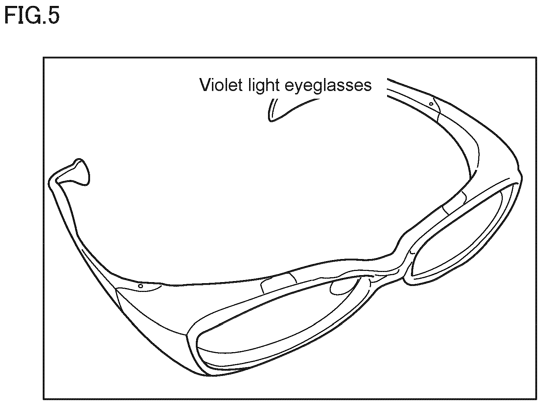

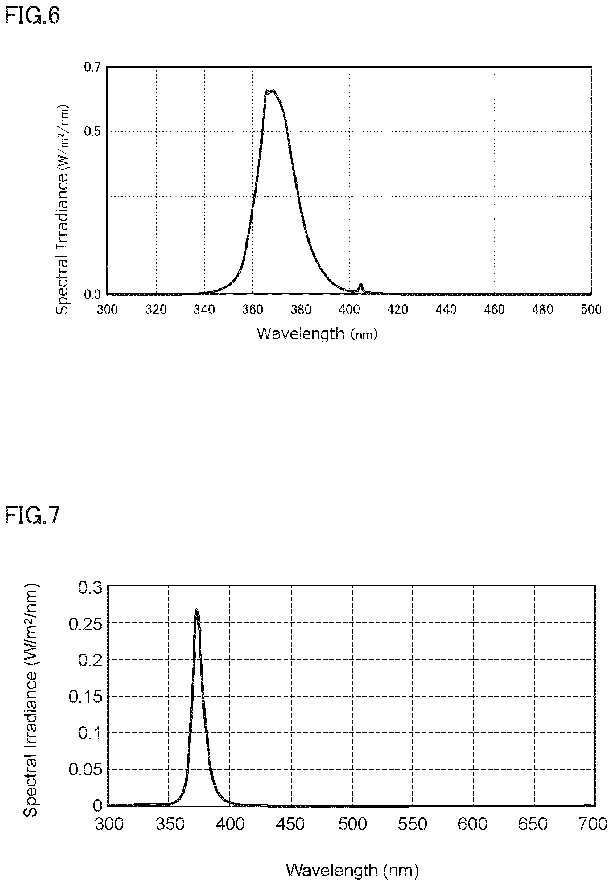

The light source used in the experimental examples of Figs. 2A and 2B, Figs. 3A and 3B, and Figs. 4A and 4B was eyeglasses with a 375-nm light-emitting diode (LED) having an irradiance with a maximum output of 310 µW/cm² attached to the frame (refer to Fig. 5). This LED exhibits the spectral wavelength shown in Fig. 7 and emits violet light defined at 360 to 400 nm. This light source includes a control unit (electronic circuit unit) that can adjust the output within a range less than or equal to the above-described maximum output. Further, the light source has a function that allows the blinking frequency to be also changed as desired, and can emit violet light within a range of 0 (direct current light that is "continuous light") to 150 Hz. The irradiation conditions in this experiment were the blinking frequencies of 10 Hz and 60 Hz. It should be noted that, in the light source used, a duty ratio of the blinking frequency was set to 50%.

As for the test method, the eyes-opening and closing test at the resting and awakening with closed eyes (awake record) was performed in the sequence below. (1) Electromyograms were taken when the eyes were closed and when the eyes were blinked at rest without wearing the eyeglasses with a light source (hereinafter, simply referred to as "eyeglasses"), and the corresponding brain waves were examined. (2) Subsequently, the brain waves were measured for five minutes in a normal state in which blinking was also naturally performed without wearing the eyeglasses. (3) Subsequently, the brain waves were measured while eyeglasses were worn and the eyes were irradiated for five minutes with violet light having a blinking frequency of 10 Hz. (4) Subsequently, the brain waves were measured while the eyes were irradiated for five minutes with the violet light changed to a blinking frequency of 60 Hz. It should be noted that the brain waves were measured by an electroencephalograph (manufactured by Nihon Kohden Corporation, EEG-1200 series model).

From the results of Figs. 2A and 2B, Figs. 3A and 3B, and Figs. 4A and 4B, when the frequency of the violet light was changed from 10 Hz to 60 Hz, the brain waves also changed from 10 Hz to 60 Hz following the change. Further, it was found that the brain waves followed the light frequency not only in the occipital lobe but also in the frontal lobe, and that the brain waves produced by the irradiation of violet light were frequency-dependent.

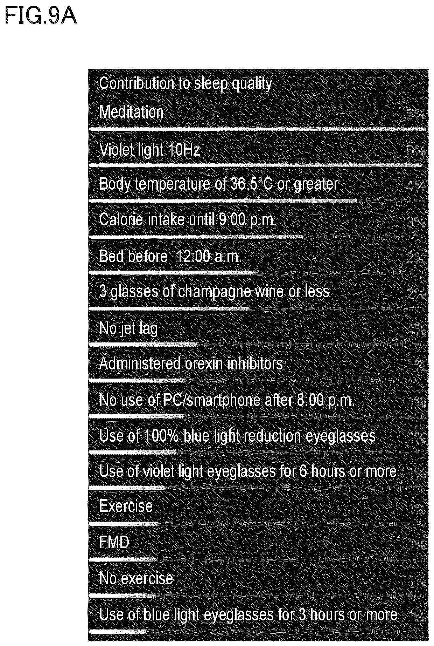

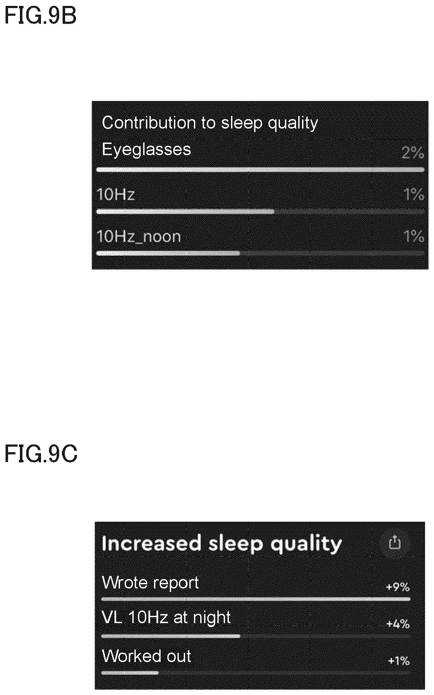

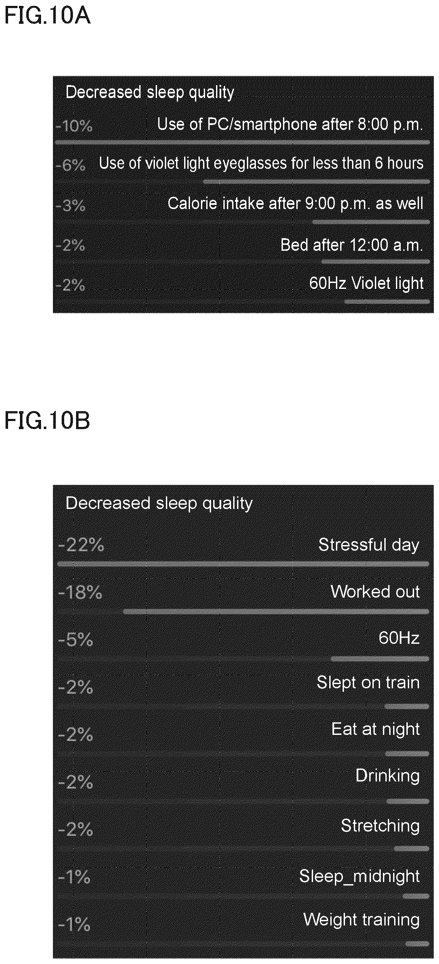

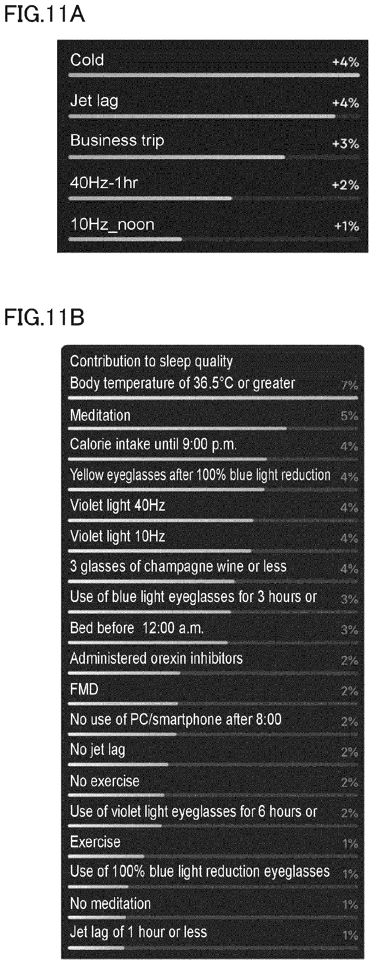

It should be noted that Figs. 9A to 9C, Figs. 10A and 10B, and Figs. 11A and 11B are data of application software when the present inventors wore the eyeglasses having a light source (Fig. 5) as necessary and led a daily life. Figs. 9A to 9C and Figs. 11A and 11B show ratios of contribution to sleep quality when the blinking frequency of the violet light was irradiated at 10 Hz, 40 Hz, and 60 Hz, and Figs. 10A and 10B show ratios of decreases in sleep quality. The irradiation of violet light at a blinking frequency produces brain waves that are the same or substantially the same as the blinking frequency, and thus it was confirmed that the brain waves thus generated had a certain effect on sleep quality.

In this experiment, the light used was light having a wavelength of 360 to 400 nm in the violet light region and the results were obtained at frequencies of 10 Hz, 40 Hz, and 60 Hz, but with the brain waves being dependent on the blinking frequency of the irradiated light, it is expected that the same phenomenon will occur at other frequencies (for example, 9 Hz, 30 Hz, or 60 Hz or greater) as well. Further, although the wavelength of the light used here is within the violet light region, it can also be expected that light within other wavelength regions will generate brain waves that are the same or substantially the same as the blinking frequency of the irradiated light. Moreover, brain waves are known to affect mental and physical actions such as emotion, motivation, memory, concentration, relaxation, mood elevation, and awakening, and thus the device can be a device capable of causing such mental and physical actions. Furthermore, action on cell activity and diseases based on brain waves can also be expected.

### (Light source)

The wavelength of the light emitted by the light source is not particularly limited, but in the above-described experiment, violet light defined at 360 to 400 nm was used. In addition to violet light, light having other wavelengths can also be expected to have the same effect and can also be used in combination with violet light. Further, because white light has also a certain effect as shown in results described later, white light may be the light emitted from the light source or may be a portion of the light.

A light source capable of an oscillation frequency of 0 (continuous light, direct current light) to 150 Hz can be preferably applied. The frequency can be adjusted in 0.5 Hz units or 1 Hz units by settings of the control unit, and light having a desired blinking frequency can be produced. Increasing the blinking frequency has the advantage that the blinking becomes less noticeable, although there are individual differences. The blinking frequency is not limited to 10 Hz or 60 Hz used in the experimental example.

The irradiance from the light source may be variable or may be constant. In the above-described experiment, a light source having an irradiance with a maximum output of 310 µW/cm² is used, but the present invention is not limited thereto. The irradiance can be configured as desired, such as, for example, within a range of 1 µW/cm² (0.01 W/m²) to 1000 µW/cm² (10 W/m²) or, for example, within a range of 0.5 µW/cm² (0.005 W/m²) to 500 µW/cm² (5 W/m²), or within a range of 0.5 to 1000 µW/cm². The brain waves that are the same or substantially the same as the blinking frequency is produced even at low irradiance, making it possible to generate predetermined brain waves by irradiation at a blinking frequency both when the eyes are opened and when the eyes are closed. Furthermore, as long as the light source has such an irradiance, the light source can be easily applied to eyeglasses and other portable irradiation devices as well, and thus can be worn in daily life as well. The occurrence of the above-described characteristic phenomenon has been confirmed even with a particularly slight amount of weak light (light having weak light sensitivity), and an effect on the brain and application to cell activity (used in the sense of including gene expression control as well) can be expected.

The light may be specified by a relative luminosity factor. It is possible to realize characteristics of the present invention even with a low relative luminosity factor, and thus irradiate violet light in a blinking manner that causes brain wave stimulation under a low relative luminosity factor and stimulate a desired site without burdening the subject.

The irradiation time of the light is preferably set as desired corresponding to purpose, and may be a short time or a long time. The light can also be made intermittent (regular intervals or irregular intervals) or continuous as desired.

The light source is preferably eyeglasses with a light source. A light source that emits blinking frequency is attached to eyeglasses that are easy to wear and do not cause discomfort on a daily basis, and thus such eyeglasses increase practicality and can be worn continuously in various situations and environments as well. Further, the light source can be a device having various light source forms in accordance with usage environment, and may be a light source installed in front of or near the face such as a desk-top light source or a light source mounted on a mobile terminal, a non-installation-type light source such as a portable light source, or an installation-type light source such as indoor lighting, a table lamp, or a dedicated device.

### (Control unit)

The control unit is a section that controls the irradiation state (continuous light or blinking frequency) of the light from the light source. The control unit may be provided with a power source (not illustrated) for supplying power to the light source, and such a power source may be a battery or may be obtained by routing a cable to a battery mounted in another position. Further, when stationary in one location, the control unit may be connected to a residential power source or the like.

The control unit preferably executes changes in irradiation conditions of the light, such as a blinking frequency, an irradiance, an irradiation time, an irradiation start time, an irradiation end time, and a blinking frequency, by transmission and reception with an isolation controller such as a mobile terminal. Such a control unit isolates and controls the various irradiation conditions described above, making it possible to set irradiation conditions suitable for producing desired brain waves and cell activity as desired to obtain a desired effect. Furthermore, this makes it possible to measure and evaluate how irradiation of a specific wavelength light at a blinking frequency affects brain waves and cell activity and to what extent the irradiation affects the mind and body, and put the results to practical use.

Further, the control unit may be provided with a controller or timer function of the light source. Examples of controller functions include functions for varying frequency and irradiance or setting the irradiation time. Furthermore, examples of timer functions include a function that allows the irradiance time of the light to be set. Such controller and timer functions may be provided in an integrated manner with an instrument or may be provided separately.

As described above, the brain wave and cell activity control device based on light stimulation according to the present invention irradiate light having a specific wavelength, such as violet light, using continuous light or a specific blinking frequency, making it possible to produce brain waves in the subject that are the same or substantially the same as a frequency of the light in the irradiation state, or induce different specific brain waves in the subject. In particular, it possible to induce specific brain waves in the subject having received the light that are the same or substantially the same as, or different from the blinking frequency of the light, and apply various stimulation to the mind, body, and brain.

### [Device for Improvement or Increase in Brain Function]

In the present invention, further studies were conducted on the effects of light stimulation by violet light and white light. As a result, it was found that, by light stimulation by irradiation of violet light or white light, improvement or prevention of depression, suppression or prevention of stress, improvement or increase in concentration, improvement or prevention of Alzheimer's disease, improvement or prevention of cognitive functions, and the like can be expected. This is the first finding of such an effect by light stimulation by violet light or white light, unprecedented in the related art, and a device for improvement, prevention, or increase in brain function according to the present invention was achieved.

A device for improvement, prevention, or increase in brain function according to the present invention is a device that improves, prevents, or increases brain function by irradiating violet light or white light onto a subject using continuous light or a specific blinking frequency, and includes a light source that emits the violet light or the white light, a light-emitting cycle control unit that sets the violet light or the white light to continuous light or a specific blinking frequency, and a light-emitting time control unit that irradiates the violet light or the white light for a specific time or a specific period. The device is used for one or two or more purposes selected from improvement or prevention of depression, suppression or prevention of stress, improvement or increase in concentration, improvement or prevention of Alzheimer's disease, and improvement of sleep.

Further, a device for improvement or prevention of brain function according to the present invention is a device that improves or prevents cognitive functions by irradiating violet light or white light onto a subject using continuous light, and includes a light source that emits the violet light or the white light, and a light-emitting time control unit that irradiates the violet light or the white light for a specific time or a specific period.

The improvement or increase in these brain functions will now be described in order. Hereinafter, violet light is denoted as "VL."

### (Improvement of depression)

Each mouse was irradiated with white light continuous stimulation (WL continuous), VL continuous stimulation (VL continuous), and VL 40-Hz frequency stimulation (VL 40Hz) to apply light stimulation. The mice used were C57BL/6 mice, 8 to 13 weeks old.

Evaluation methods of depression include a tail suspension test (TST, https://www.jove.com/video/3769/?language=Japanese) in which the mouse is held by the tail and the degree to which the mouse struggles is quantified, and a forced swim test (FST, https://www.jove.com/video/3638/the-mouse-forced-swim-test) in which the mouse is forcibly made to swim in a small pool and the degree to which the mouse struggles is measured, and evaluation was carried out this time by TST. It should be noted that the degree to which a mouse "gives up" is an index of depression in the depressed state, and examples of non-patent documents on evaluation include "Journal of Visualized Experiments, January 2012, 59, e3638, pages 1 of 5," "Journal of Visualized Experiments, January 2012, 59, e3769, pages 1 of 5," and "Neuron 53, 337-351, February 1, 2007, a2007, Elsevier Inc., 337."

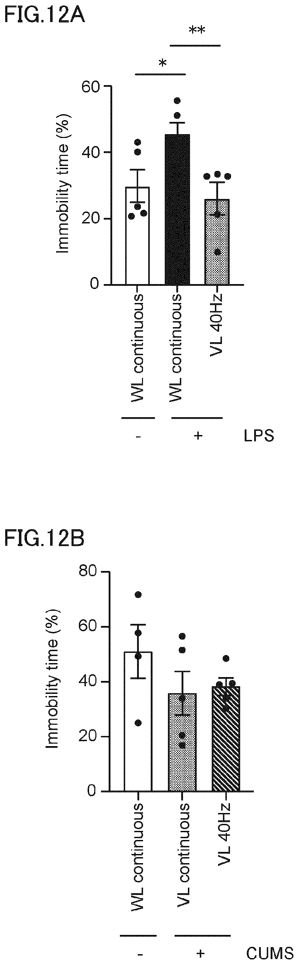

Fig. 12A is an evaluation result of inducing depression by using lipopolysaccharide (LPS) and improving depression by VL 40-Hz frequency stimulation. LPS was administered to the mice to induce an acute inflammatory depressive state. VL 40-Hz frequency stimulation was applied for a total of eight days before and on the day of LPS administration, and depression was evaluated by the tail suspension test (TST). The reference used for the LPS-based depression inducing experiment was https://www.nature.com/articles/s41598-019-42286-8. As shown in Fig. 12A, a reduction in symptoms of depression by VL 40-Hz frequency stimulation was observed. Immobility times of TST were measured by ANY-maze (Video Tracking System / Muromachi Kikai Co., Ltd.), statistically significant differences between a control group, an LPS group, and an LPS + 40-Hz frequency stimulation group were verified by GraphPad Prism 8.0 software, and the results are shown in Figs. 12A and 12B. It should be noted that "^{∗}" indicates p < 0.05 and "^{∗∗}" indicates p < 0.01.

Fig. 12B is an evaluation result of inducing depression by using chronic unpredictable mild stress (CUMS) and improving depression by VL continuous or 40-Hz frequency stimulation. The reference used for the experiment inducing depression by CUMS was https://www.sciencedirect.com/science/article/pii/S0149763418304378?via%3Dihub. Mice were given various relatively slight stresses on a daily basis for a long period of time (seven weeks) to induce a chronic psychological depressive state. During that time, VL continuous stimulation or VL 40-Hz frequency stimulation was applied, and depression was evaluated by TST. As shown in Fig. 12B, depression symptoms were improved by VL continuous stimulation or VL 40-Hz frequency stimulation.

### (Suppression of stress and increase in concentration)

The suppression of stress and the increase in concentration by VL were analyzed. With regard to stress data, the brain wave (electroencephalogram; EEG) measuring method and evaluating method were the methods described in the non-patent document "Sensors 2018, 18 (12), 4477" (https://doi.org/10.3390/s18124477, https://www.mdpi.com/1424-8220/18/12/4477/htm).

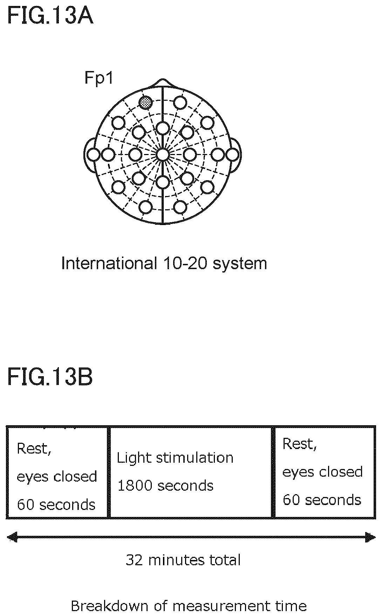

In the experiment, using VL and white light, light stimulation was applied with blinking and continuous light. Measured brain waves were analyzed by filtering the waves as a pretreatment, calculating power spectra by fast Fourier transform (FFT), and conducting a significance test based on the result. The total number of subjects was 162, the measurement location was the left prefrontal cortex Fp1 (international 10-20 system) shown in Fig. 13A, and the measurement time was 30 minutes (1800 seconds) with a rest time of one minute before and after the measurement time as shown in Fig. 13B. The light stimulations at the time of measurement were white light continuous stimulation, white light frequency stimulation, VL continuous stimulation, and VL frequency stimulation. The VL stimulation was applied with the VL eyeglasses shown in Fig. 5, and the white light stimulation was applied with a frequency stimulation of 40 Hz. A simple electroencephalograph (sampling frequency: 512 Hz, MindWave mobile BMD version, Neurosky Inc.) was used as the measuring device.

The analysis was performed in accordance with the following procedure. Light stimulation was applied to 81 persons using VL stimulation and 81 persons using white light stimulation under eight conditions (seven conditions per light stimulation and one condition of light-off) to measure the brain waves. The light stimulation time was 30 minutes (1800 seconds) as shown in Fig. 13B. The obtained measurement data were subjected to noise removal (filtering enabling 1 to 70 Hz) and power spectrum calculation (Hilbert transform, spline interpolation) to calculate a one-sided power spectrum. This procedure was served as standard.

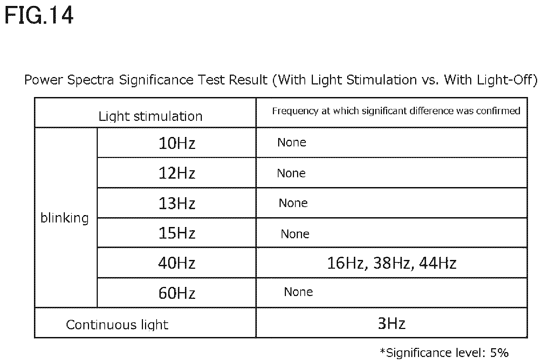

A significant difference test was performed to analyze the frequency components of the brain waves affected by light stimulation. Specifically, a bilateral two-sample t-test (significance level: 5%) was performed on healthy males and females. The significance test was performed by comparing each of the seven conditions of frequency stimulation and continuous light stimulation with the continuous light-off state for a 1-Hz power spectrum (average of the subjects). It should be noted that the power spectra of 2 to 45 Hz were also compared. The significance test results of the power spectra (with light stimulation vs. with light-off) are shown in Fig. 14. As shown in the results of Fig. 14, in the frequency stimulations of 10 Hz, 12 Hz, 13 Hz, 15 Hz, 40 Hz, and 60 Hz, the power spectra were significantly large at 16 Hz, 38 Hz, and 44 Hz in the case of the 40-Hz frequency stimulation (significance level: 5%). On the other hand, no significant difference was confirmed in the frequency stimulations of 10 Hz, 12 Hz, 13 Hz, 15 Hz, and 60 Hz. Further, with continuous light, the power spectrum of 3 Hz was significantly small.

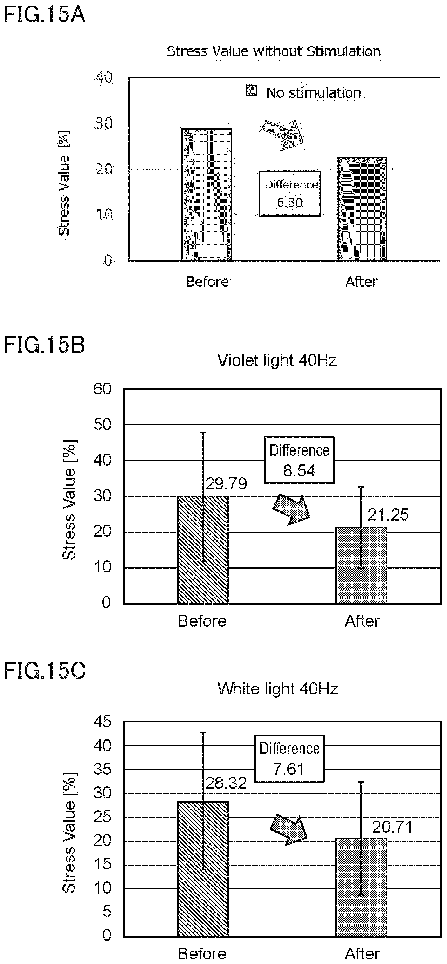

Next, the suppression of stress by 40-Hz frequency stimulation was examined. A white light 40-Hz frequency stimulation and a VL 40-Hz frequency stimulation were applied to healthy males and females before, during, and after a task. Noise was removed and power spectra were analyzed from the brain waves measured using a simple electroencephalograph, stress values (%) were verified, and power spectra were calculated. The analysis means were the means based on https://www.mdpi.com/1424-8220/18/12/4477. The results are shown in Figs. 13A and 13B. Fig. 15A shows stress values before and after task without stimulation, Fig. 15B shows stress values before and after VL 40-Hz frequency stimulation, and Fig. 15C shows stress values before and after white light 40-Hz frequency stimulation. It was found that stress was reduced by 40-Hz frequency stimulation compared to before stimulation and after task without stimulation.

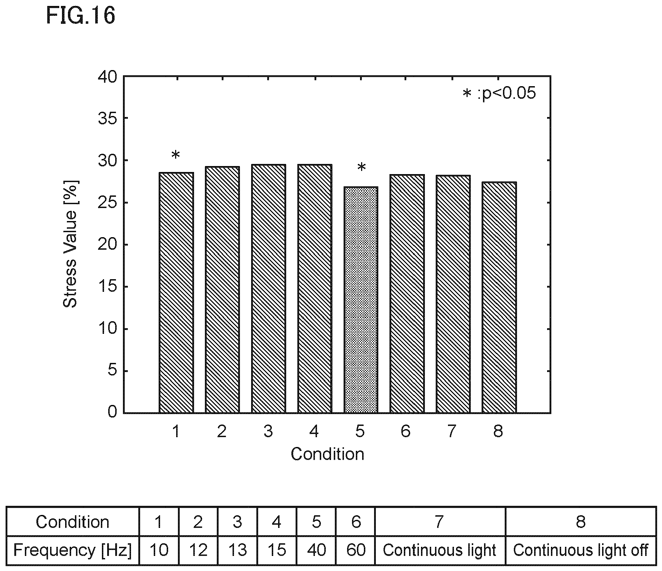

Fig. 16 is a graph showing an average value of stress values for stimulation under each condition ("^{∗}" indicates p < 0.05). Healthy males and females were given VL frequency stimulations of 10 Hz, 12 Hz, 13 Hz, 15 Hz, 40 Hz, and 60 Hz, and continuous light stimulation. Noise was removed and power spectra were analyzed from the brain waves measured using a simple electroencephalograph, stress values (%) were verified. From this result, stress was reduced by the 10-Hz and 40-Hz frequency stimulations.

The subjects were asked to evaluate, on a five-point scale, whether or not they could read an article during the light stimulation experiment, and the possibility of improving intellectual productivity was examined. With VL frequency stimulation, the results were "Read very well: 5.9%," "Read well: 41.2%," "Read as usual: 47.1%," "Did not read very well: 5.9%," and "Could not read at all: 0%." On the other hand, with white light frequency stimulation, the results were "Read very well: 0%," "Read well: 5.9%," "Read as usual: 70.6%," "Did not read very well: 23.5%," and "Could not read at all: 0%."

### (Improvement of Alzheimer's disease)

With regard to the improvement effect on Alzheimer's disease, white light continuous stimulation (WL continuous), white light 40-Hz frequency stimulation (WL 40Hz), VL continuous stimulation (VL continuous), and VL 40-Hz frequency stimulation (VL 40Hz) were each applied to the mice and evaluated.

For Alzheimer's disease, tau is known as a causative gene of Alzheimer's disease in humans. While it is known that mutations in this gene cause Alzheimer's disease, the tau gene mutation S301P was discovered in 1999 as a mutation in a familial genetic disease of Parkinson's disease or frontotemporal dementia. In the experiment, an evaluation was conducted using mice including human S301P tau. In fact, phosphorylated tau is a diagnosis of Alzheimer's disease, and is detected using an antibody called AT8 (pSer202/Thr205). The first paper that evaluated Alzheimer's disease by using S301P mice was https://www.ncbi.nlm.nih.gov/pubmed/17270732, which was applied in this experiment.

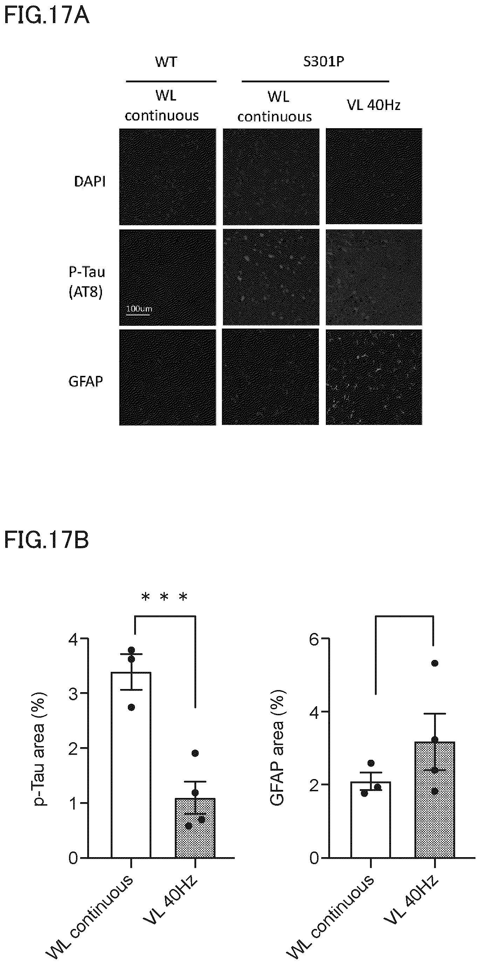

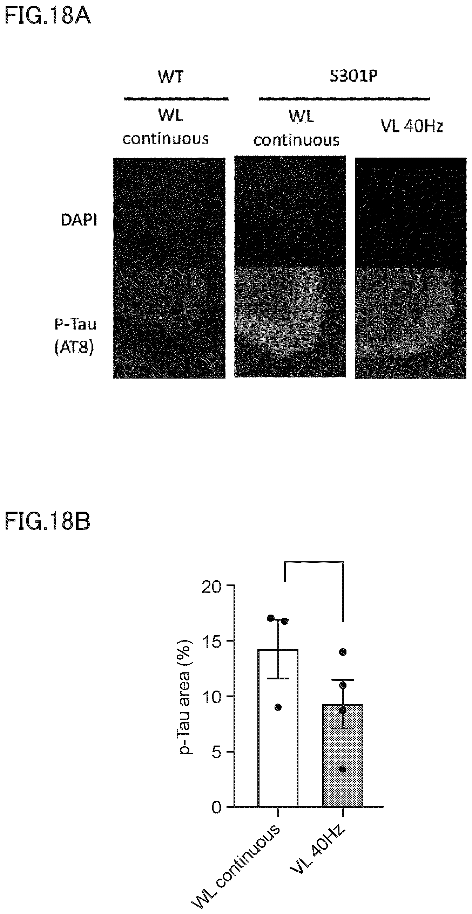

Figs. 17A and 17B and Figs. 18A and 18B are results showing a decrease in phosphorylated tau by 40-Hz frequency stimulation. Fig. 17A and Fig. 18A are measurement images of DAPI, p-Tau, and GFAP, and Fig. 17B and Fig. 18B are graphs of the area percentage of GFAP obtained by analysis of the images. Three-month-old S301P mutant mice were given white light continuous stimulation, white light 40-Hz frequency stimulation, and VL 40-Hz frequency stimulation for four weeks, respectively. Phosphorylated tau (Ser202, Thr205) was detected using AT8 antibodies. The VL 40-Hz frequency stimulation decreased the accumulation of phosphorylated tau in the hippocampus (graph on left side of Fig. 17B), decreased the accumulation of phosphorylated tau in the CA3 region (graph of Fig. 18B), and increased GFAP in astrocytes (graph on right side of Fig. 17B).

### (Improvement of cognitive functions)

With regard to the improvement effect on cognitive functions in aged mice, white light continuous stimulation (WL continuous), white light 40-Hz frequency stimulation (WL 40Hz), VL continuous stimulation (VL continuous), and VL 40-Hz frequency stimulation (VL 40Hz) were each applied to the mice and evaluated.

With regard to a cognitive memory experiment, memory is evaluated using two types, fear memory and spatial memory, but fear memory is evaluated by the time of absence of movement upon freezing after applying an electric shock. While a mouse freezes immediately after an electrical stimulation is applied, when the mouse is placed in a machine that applies an electric shock the next day and does not have any memory of the previous day, the freezing time decreases (reference: https://www.ncbi.nlm.nih.gov/books/NBK5223/). On the other hand, for spatial memory, when a mouse is exposed to strong light while placed on a white disk, a nocturnal mouse tries to escape to a dark place. There are 20 holes in the disk, and 19 are closed, but escape can be made only through one hole. Mice are trained to remember the location of that one hole over a period of six days. A method of evaluating one day later and one week later to what extent the location of the hole can be memorized was used (reference: https://www.nature.com/protocolexchange/protocols/349Reiserer).

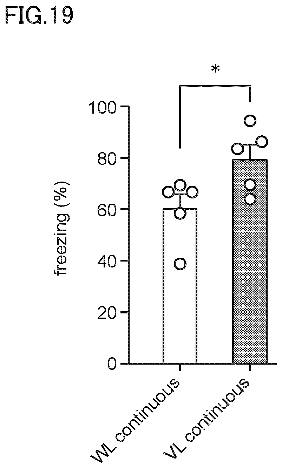

Fear memory was evaluated by a contextual fear conditional (CFC) test. After white light continuous stimulation or VL continuous stimulation was applied to 64-weak-old aged mice for seven weeks, fear memory was evaluated by the CFC test. As shown in Fig. 19, it was confirmed that a CFC freezing score was improved by VL continuous stimulation.

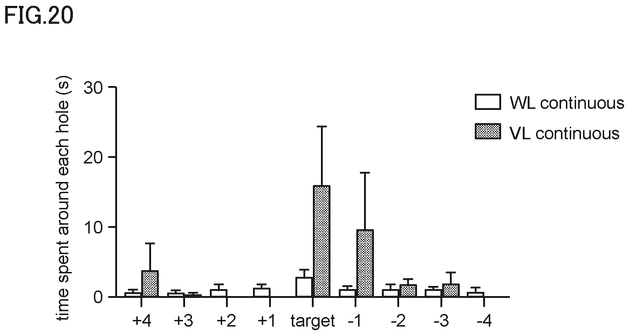

A Barnes maze test was conducted. After white light continuous stimulation or VL continuous stimulation was applied to 64-week-old aged mice for 11 weeks, spatial memory was evaluated by a Barnes maze. The maze was memorized over a period of six days by training, and a probe (memory test) was performed to measure long-term memory seven days later. As a result, as shown in Fig. 20, the score of the aged mice was improved by VL continuous stimulation. The result was verified by two-way analysis of variance (ANOVA), and the P value was < 0.05.

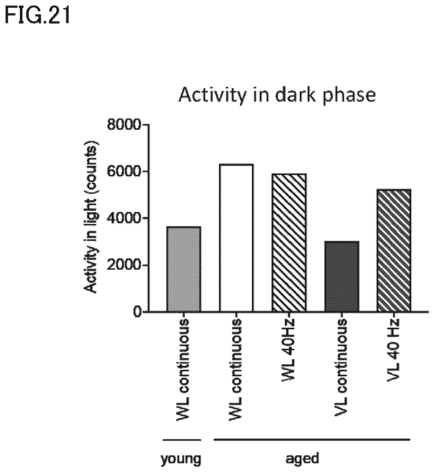

Activity was evaluated. Activity was evaluated using a running wheel after applying VL continuous stimulation to 75-week-old aged mice for 12 weeks. As shown in Fig. 21, an activity score in the aged mice was improved by each of the white light 40-Hz frequency stimulation, the VL continuous stimulation, and the VL 40-Hz frequency stimulation.

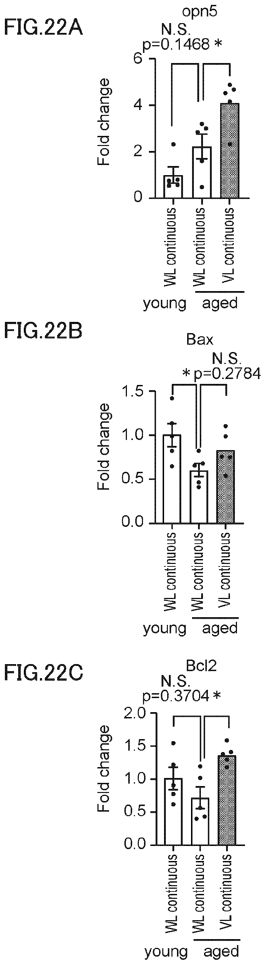

Figs. 22A to 22C, Figs. 23A and 23B, and Figs. 24A to 24C show changes in gene expression by VL continuous stimulation. VL continuous stimulation was applied to 67-week-old aged mice for 13 weeks. The changes in gene expression of the mice and the mice under white light continuous conditions of the 15-week-old young mice (young) and the 67-week-old aged mice (aged) were compared. "^{∗}" indicates p < 0.05, "^{∗∗}" indicates p < 0.01, and "^{∗∗∗}" indicates p < 0.001.

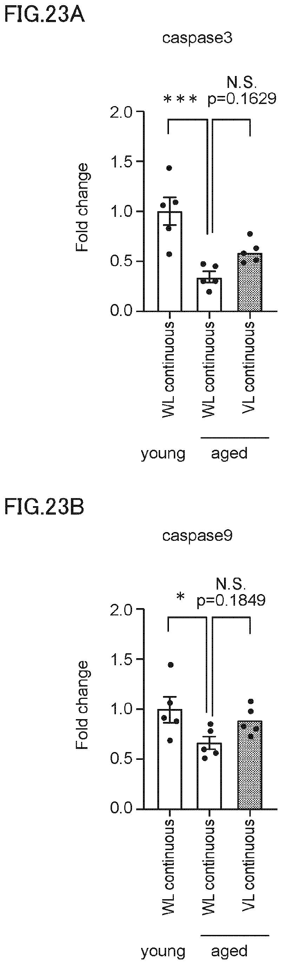

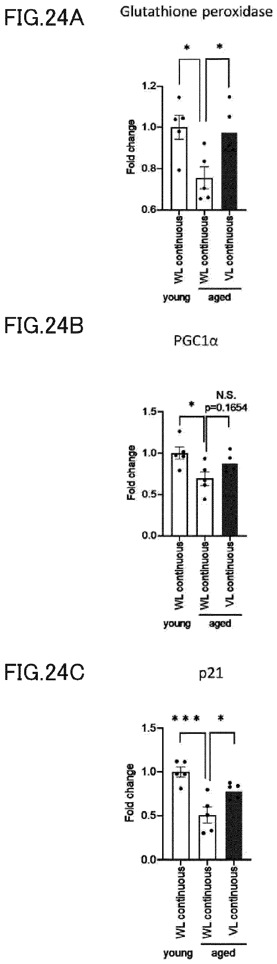

As shown in Figs. 22A to 22C, Figs. 23A and 23B, and Figs. 24A to 24C, by VL continuous stimulation, rises occurred in the opn5 gene expression (refer to Fig. 22A), the apoptosis-related gene Bax (refer to Fig 22B), the Bcl2 gene expression (refer to Fig. 22C), the apoptosis-related gene Caspase3 (refer to Fig. 23A), the Caspase9 gene expression (refer to Fig. 23B), the oxidative stress- and mitochondria-related glutathione peroxidase and PGC1α gene expression (refer to Figs. 24A and 24B), and the cell cycle- and cell aging-related p21 gene expression (refer to Fig. 24 C).

At the present time, the fact that VL stimulation is effective for brain function and cell activation is considered to be based on the principle of mediation by a VL-specific photoreceptor (neuropsin) called OPN5. References showing that OPN5 is a VL neuropsin in the retina include https://journals.plos.org/plosone/article?id=10.1371/journal.pone.0026388. However, the light stimulation of the present invention described above is not necessarily passed through the eyes, and conceivably penetrates the skull and acts directly on the brain (refer to https://www.cell.com/current-biology/fulltext/S0960-9822(14)00603-4). In the present invention, from the result of Figs. 22A to 22C described above as well, the gene expression of OPN5 is considered to rise by VL irradiation.

As shown by the results above, according to the device for improvement or increase in brain function according to the present invention, it is possible to improve or prevent depression, suppress or prevent stress, improve or increase concentration, improve or prevent Alzheimer's disease, improve or prevent cognitive functions, improve sleep, and the like by a device that irradiates violet light or white light onto a subject.

## Claims

1. A brain wave and cell activity control device, based on light stimulation, that controls brain waves or cell activity by irradiating light having a specific wavelength onto a subject using continuous light or a specific blinking frequency, the brain wave and cell activity control device comprising:
a light source that irradiates the light having a specific wavelength using continuous light or a specific blinking frequency; and
a control unit that controls an emission of light that induces specific brain waves, the brain waves of the subject having received the light being the same or substantially the same as, or different from a frequency of the light in an irradiation state.

2. The brain wave and cell activity control device according to claim 1, wherein
the light is violet light.

3. The brain wave and cell activity control device according to claim 1 or 2, wherein
the irradiation state of the light is a continuous light or a blinking frequency greater than 0 Hz and less than or equal to 150 Hz.

4. The brain wave and cell activity control device according to any one of claims 1 to 3, wherein
the light is irradiated at an irradiance within a range of 0.5 to 1000 µW/cm².

5. The brain wave and cell activity control device according to any one of claims 1 to 4, wherein
the control unit changes and executes irradiation conditions of the light, such as the irradiation state (including continuous light or blinking frequency), an irradiance, an irradiation time, an irradiation start time, an irradiation end time, and continuous light or blinking frequency, by transmission and reception with an isolation controller such as a mobile terminal.

6. The brain wave and cell activity control device according to any one of claims 1 to 5, wherein
the light source is a light source installed in front of or near a face, such as eyeglasses with a light source, a desk-top light source, or a light source mounted on a mobile terminal.

7. The brain wave and cell activity control device according to any one of claims 1 to 5, wherein
the light source is a non-installation-type light source such as a portable light source, or an installation-type light source such as indoor lighting, a table lamp, or a dedicated device.

8. A brain wave and cell activity control method, based on light stimulation, of controlling brain waves or cell activity by irradiating light having a specific wavelength onto a subject using continuous light or a specific blinking frequency, the brain wave and cell activity control method comprising:
controlling an emission of light that induces specific brain waves, the brain waves of the subject having received the light being the same or substantially the same as, or different from of the continuous light or the specific blinking frequency.

9. A device for improvement, prevention, or increase in brain function by irradiating violet light or white light onto a subject using continuous light or a specific blinking frequency, the device comprising:
a light source that emits the violet light or the white light;
a light-emitting cycle control unit that sets the violet light or the white light to continuous light or a specific blinking frequency; and
a light-emitting time control unit that irradiates the violet light or the white light for a specific time or a specific period,
the device being used for one or two or more purposes selected from improvement or prevention of depression, suppression or prevention of stress, improvement or increase in concentration, improvement or prevention of Alzheimer's disease, improvement of sleep, and the like.

10. A device for improvement or prevention of brain function that improves or prevents cognitive functions by irradiating violet light or white light onto a subject using continuous light, the device comprising:
a light source that emits the violet light or the white light; and
a light-emitting time control unit that irradiates the violet light or the white light for a specific time or a specific period.
